# EUROPEAN PATENT SPECIFICATION

(11) **EP 1 996 950 B1**
(45) Date of publication and mention of the grant of the patent: **04.11.2009**
(21) Application number: 07705261.1
(22) Date of filing: 23.02.2007
(51) Int. Cl.: G01N 33/68

(54) **INFECTIVITY ASSAY**
INFEKTIÖSITÄTSTESTVERFAHREN
DOSAGE D'INFECTIVITÉ

(30) Priority: 24.02.2006 GB 0603775
(43) Date of publication of application: 03.12.2008
(73) Proprietor: Health Protection Agency, Salisbury Wiltshire SP4 0JG (GB)
(72) Inventor: HESP, Richard, Wiltshire SP4 0JG (GB); SUTTON, J., Mark, Wiltshire SP4 0JG (GB); ALEXANDER, Frances, Wiltshire SP4 0JG (GB); KIRBY, Elizabeth, Wiltshire SP4 0JG (GB); RAVEN, Neil, Wiltshire SP4 0JG (GB)
(74) Representative: MacLean, Martin Robert
(86) International application number: PCT/GB2007/000630
(87) International publication number: WO 2007/096633

(56) References cited:
- WO-A-02/24871
- FEVRIER BENOIT ET AL: "Cells release prions in association with exosomes" PROCEEDINGS OF THE NATIONAL ACADEMY OF SCIENCES OF THE UNITED STATES OF AMERICA, vol. 101, no. 26, 29 June 2004 (2004-06-29), pages 9683-9688, XP002432629 ISSN: 0027-8424
- PORTO-CARREIRO ET AL: "Prions and exosomes: From PrPc trafficking to PrPsc propagation" BLOOD CELLS, MOLECULES AND DISEASES, LAJOLLA, US, vol. 35, no. 2, September 2005 (2005-09), pages 143-148, XP005067364 ISSN: 1079-9796
- SAFAR JIRI G ET AL: "Diagnosis of human prion disease." PROCEEDINGS OF THE NATIONAL ACADEMY OF SCIENCES OF THE UNITED STATES OF AMERICA 1 MAR 2005, vol. 102, no. 9, 1 March 2005 (2005-03-01), pages 3501-3506, XP002432630 ISSN: 0027-8424

## Description

The present invention relates to methods for promoting the infection of a cell culture line with a TSE agent to generate an infected cell line, preferably a stably transfected cell line. The present invention also relates to uses of the infected cell line for the detection of a TSE agent and for the diagnosis of TSE disease, and for the identification of therapeutic agents suitable for treating TSE disease.

Transmissible Spongiform Encephalopathies (TSEs) are a family of related neurodegenerative disorders that includes Creutzfeldt-Jacob Disease (CJD) and Kuru in humans, Bovine Spongiform Encephalopathy (BSE) in cattle and Scrapie in sheep. TSEs present as a familial, sporadic or iatrogenic disease a frequency of approximately 1 case per million population (Will, 1993). However, the emergence of a variant form of Creutzfeldt-Jacob Disease (vCJD) in the UK in the 1990s - potentially due to consumption of BSE-infected meat products (Bruce, *et al*, 1997; Hill *et al,* 1997) - has raised the possibility of much higher levels of CJD incidence.

The transmissible nature of TSE diseases is due to a unique infectious entity, termed a prion, which is proposed to have no nucleic acid component (Prusiner 1982). TSE diseases are characterised by the formation of mutant forms (PrP^{Sc}) of the normal cellular prion protein (PrP^{c}). The mutant prion, PrP^{Sc}, is highly resistant to physical and chemical degradation. In particular, PrP^{Sc} is highly protease resistant, as compared with the normal prion, PrP^{c}.

Protease resistant prions may arise heritably as a result of mutations (ie. familial TSE disease), or sporadically by an as yet undefined mechanism (ie. sporadic TSE disease). In addition, protease resistant prions may arise by "infection" following exposure of normal prion to exogenous resistant prion - for example by transfusion of infected blood, by tissue implantation/transplantation, or by exposure to contaminated medical products/ surgical instruments (ie. iatrogenic TSE disease).

Rigorous practices have been adopted in the agricultural and meat-rendering industries to reduce the risk of cross contamination between BSE infected carcasses and meat that is destined for human consumption. However, BSE infected animals can still be unintentionally processed in abattoirs, especially if the animal is in the early stages of the disease and therefore undetected as an infected TSE host. There is also a considerable risk in disposal of known BSE infected material, particularly if the equipment used in the disposal operation is then reused in normal rendering practices without adequate sterilisation.

The proteolytic resistant nature of the PrP^{SC} prion means that it is largely unaffected by standard methods of sterilisation. Thus, the combination of exceptional stability, diverse routes of transmission and problems with diagnosis and detection has important public health consequences.

In particular, it has proved difficult to define the level of risk of transmission of human prion disorders via surgery, transplant or transfusion. There are well-documented cases of transmission via neurosurgery (Bernoulli *et al* 1977; Brown *et al,* 2000), transplant of dura mater (Anon 1987; Brooke *et al,* 2004), and use of human growth hormone (Brown *et al*, 2000; Swerdlow *et al*, 2003), all involving sporadic forms of the disease. The possibility of transmission via routine surgery also exists, and some studies have suggested that an increased rate of sporadic CJD is increased with the number of surgical events that an individual may have undergone (Collins *et al,* 1999; Ward *et al,* 2002).

The long incubation period of the disease at either a pre-clinical or sub-clinical level (as reviewed by Hill and Collinge, 2003) means that it is currently not possible to estimate the number of people carrying the disease. All these carriers, irrespective of whether they go on to develop symptoms, may be able to transmit the disease to other people, particularly people of a more susceptible genotype, via any of the routes described above.

The emergence of vCJD has made estimation of the levels of risk far more difficult as certain aspects of its presentation differ significantly from that of the more traditional forms. By way of example, the vCJD agent is found at much higher levels in lymphoid tissue such as tonsils and the appendix (Hilton *et al*, 2002; Joiner *et al*, 2002) and the peripheral nervous system (Herzog *et al*). Both these features suggest that the iatrogenic transmission of vCJD is more likely than in traditional forms of CJD, due to the much higher numbers of surgical practices involving these tissues when compared to surgery of the central nervous system (although prion material has been found in skeletal muscle from sporadic CJD patients - Glatzel *et al,* 2003). There is now goods evidence that vCJD, at least, may be transmissible by blood transfusion (Llewelyn *et al,* 2004; Peden *et al,* 2004), and this further increases the risk associated with routine surgery and the handling of contaminated surgical instruments.

The key areas of uncertainty result in part from the absence of suitable detection and/ or diagnosis tools for investigating TSE diseases, resulting in significant problems for the management of both human and animal TSE diseases.

A number of methods have been developed for post-mortem analysis of brain material from cattle suspected to be infected with BSE, and these have been adapted for diagnosis of the chronic wasting disease in deer. However, there are currently no methods for ante-mortem tests in animals.

Detection and diagnosis of all forms of human TSE disease is very difficult because there is no validated detection method currently available. Diagnosis of people suspected to be infected with a TSE disease is therefore very difficult, with neurophysiological measurements and behavioural scores as the primary diagnostic criteria, with support from tonsil biopsy, and confirmation only post mortem. In this regard, the tonsil biopsy - whilst providing useful supporting data for diagnosis - is not currently definitive and is not conducive to diagnostic use for screening purposes. In particular, the usefulness of the tonsil biopsy is limited to detection of vCJD (since the vCJD prion agent is not found in significant levels in tonsil tissue in familial, sporadic or iatrogenic forms of CJD).

Identification of TSE strain type continues to be an important area of research, since the identification of the origins of a TSE strain can help identify the origins of the agent, and may provide valuable evidence with respect to the route of infection. Known methods for identifying the origins of different TSE strains include biochemical methods (which are still not entirety proven) and bioassays using multiple animal strains to define the relative infectivity of the isolated strain. These studies are time consuming, costly, and working with potentially large numbers of animals has ethical implications.

Thus, the availability of a suitable method for the detection and culture of TSE strains would be extremely valuable, both in terms of being able to reduce dependence on animal testing but also in terms of reducing the cost and time involved for diagnosis.

Aside from their diagnostic potential, cell lines infected transiently or stably with TSE agent would be extremely useful models for screening for drugs that are able to interfere with the pathogeneses of the infection and to probe the underlying processes leading to cell invasion and death.

Thus, the development of a TSE infectable cell line would be particularly useful for developing therapies for TSE diseases. While a number of cell models have been suggested, only a very limited number of such models have been established that are capable of stably propagating TSE strains. The cell lines described in the literature are severely limited by the range of infectious agents that can be used. In more detail, only a limited number of cell lines can be infected by a very limited range of TSE agents.

The only published combinations are N2a, SMB or GT7 cell lines infected with RML scrapie (see, for example, WO 02/059615 and WO 03/081249, which describe N2a/ RML infectious systems).

A single publication (Arjona *et al*, 2004) cites the infection of GT1 and N2a cell lines with human prion agents (sporadic CJD). Whilst these results remain to be substantiated, this document describes infection using very defined CJD strains and does not address the broader issue of why certain cell types can only be infected by certain agents.

A number of other groups have tried to infect cell lines with BSE, CJD, other strains of scrapie and other TSE agents, but have been unsuccessful. The reasons for failure to be able to generate cellular infection models for other TSE strains (such as BSE and vCJD) are unclear, but may relate to the need to maintain a fine balance between stable propagation of the infectious agent and cell death, as observed during TSE infection.

WO 02/24871 describes a method for *in vitro* propagation of the agent responsible for TSE.

Fevrier B. et al., PNAS Vol. 101 (26), 29 June 2004, pages 9683-9688 reports that cells release prions in association with exosomes.

There is therefore a need in the art for a cell infection model that is able to support the infection and/ or propagation of a range of TSE agents/ strains, and therefore be useful for diagnosis of TSE diseases and/ or for screening of potential therapeutics.

The present invention meets this need in the art by providing a method for infecting a target cell with a TSE agent, as defined in the claims.

The TSE agent is preferably one selected from the group consisting of CJD, vCJD, sporadic CJD, familial CJD, iatrogenic CJD, BSE, ovine BSE, and CWD. In a further embodiment, the TSE agent may be scrapie.

In a preferred embodiment, the source of the TSE agent is selected from the group consisting of lymphatic tissues (eg. tonsil, appendix, rectal tonsil), neuronal tissues (eg. brain, central nervous system), and blood (eg. lymphocytes). In one embodiment, said tissues/ blood may be of human origin. In another embodiment, said tissues/ blood may be of bovine origin. In further embodiments, said tissues/ blood may be of ovine/ deer origin.

The method of the present invention allows cells to resist cell death during the early stages of exposure to infectious TSE agent, thus allowing the cells to stably propagate the agent during subsequent growth and cell division. In one embodiment, the infected cell lines of the present invention demonstrate stability for at least 2 weeks, typically for at least 4 weeks. In a preferred embodiment, the infected cell lines demonstrate stability for at least 2 months, preferably for at least 3 months, and more preferably for at least 4 months.

The method of the present invention provides means of allowing the infection of novel combinations of cell lines and TSE agents, thereby overcoming the. "species barrier". In particular, the present method is suitable for the establishment of diagnostic models for CJD.

The method of the present invention has a number of useful applications for the diagnosis of TSE diseases and for the screening of potential drug candidates, as described in more detail below.

In one embodiment of the present invention, the source of infectious material - for example, infected animal brain, tissue or blood (eg. BSE-301V infectious VM mouse brain) - is mixed with an intermediate (ie. donor) membrane preparation prior to ultimate infection of a target cell. This mixing is carried out in a specific mixing step, and preferably in the substantial absence of whole cells, prior to subsequent target cell infection. This mixing step helps to improve the compatibility between the TSE type and the ultimate target cell.

In one embodiment, the infectious agent is not purified prior to mixing with the donor membrane preparation. In this regard, it is preferred that the infectious agent is present as part of a membrane preparation (ie. the infectious agent is preferably purified to remove whole cells but membrane components are retained).

By introducing the infectious agent into an intermediate (ie. a donor) membrane, the agent becomes integrated into a contiguous membrane in which it is presented favourably for subsequent cell infection to occur. In this regard, it has been postulated that one mechanism for cell meditated conversion of PrP^{c} by PrP^{sc} involves the intact PrP^{sc} jumping to the target cell, including its GPI anchor, and then acting in cis on other PrP^{c} molecules (Hooper, 2002). By pre-inserting the PrP^{sc} into an intermediate (ie. donor) membrane, the method of the present invention negates the apparent requirement of direct cell contact between infectious agent and target cell, and increases the overall efficiency of the process.

In one embodiment, cell-specific co-factors, such as caveolae-like domains, are present in the intermediate (ie. donor) membrane preparations. When present, these co-factors may aid the initial stages of infectivity, and may separately act as chaperones to facilitate endocytosis and trafficking into various intracellular compartments, and affect different cellular processes. For instance, transportation of infectious agent into lysosomes could result in inhibition or overloading of that organelle which in turn impairs the ability of the cell to then remove/ degrade further infectious molecules. Transportation of the infectious agent into these compartments may therefore help to ensure that the cell is stably infected and that subsequent generations also remain infected.

In one embodiment, the intermediate (ie. donor) membranes of the present invention (including the integrated infectious agent) exert a direct effect on the target cell. In this regard, in use of the present invention, said membranes (eg. in the form of microsomes or exosomes) are transported into the target cells using endocytic pathways. These pathways may lead to the target cell's degradative pathways (eg. lysosomes), becoming overloaded, thereby allowing the infectious agent to become established in the target cell.

Cells are typically exposed to the infectious agent for 24-96 hours. The exact time is dependent on the concentration of inoculum, which may necessitate longer incubations for 'weaker' preparations, or conversely shorter times where there is considerable cell death upon exposure. The inoculum is removed by media changes and the extent of infection is monitored by immunological methods for detection of PrP^{sc}, such as those described in the examples below. After approximately 1-2 weeks post exposure, populations of transiently infected cells either die or become stably infected. These cell lines have a lifespan of between about 3 to 4 months and are able to be split as and when required without loss of the PrP^{sc} or their distinctive morphology.

A wide range of different types of membrane preparations may be employed as the 'donor' in the present invention. Preferably, the donor membrane is present in the form of a substantially cell-free preparation (ie. it does not include whole cells containing intact nuclei). Preferably, the donor membrane is substantially prion-free (especially PrP^{sc}-free). Preferably, the donor membrane is a cell-type that is different from the infectious agent (ie. TSE) source material cell-type.

Centrifugation forms the basis of most methods for enrichment of membrane fractions - eg. microsomal or exosomal membranes. By way of example, a total crude microsomal fraction may be prepared using the relatively simple differential based approach, which allows the isolation of membranes comprising vesicles derived from all types of cellular membranes - including the plasma membrane, endoplasmic reticulum and golgi, for example. Many of the intracellular bodies from which the above membranes are derived have been associated with PrP^{sc}, the putative infective agent. This type of preparation, therefore, provides a source of membranes that is associated with both the extracellular and intracellular forms of the prion agent at various stages of processing, together with any necessary co-factors required for infectivity.

Alternatively, enrichment fractions may be employed, such as enriched plasma membrane or endosomal membrane preparations. In this regard, plasma membrane and endosomal membranes are believed to be one of the main sites for prion propagation.

A range of conventional enrichment techniques may be employed for refinement of the microsomal fraction - for example, density gradient centrifugation and/ or temperature dependent phase separation using Triton X-114 (Bordier C, 1981).

For example, temperature dependent phase separation using Triton X-114 facilitates the isolation of detergent-resistant lipid 'raft domains', or caveolae-like domains, which have been shown to contain a high level of infectivity (Safar *et al.,* 1990). These domains have also been shown to be important in the conversion of PrP^{c} to PrP^{sc} (Taraboulos, 1995).

The mixing of infectious material and intermediate (ie. donor) cell membrane preparations may be carried out by any conventional method - for example, methods involving simple mixing, shearing of infectious material with the membrane preparation, and the generation of fused membrane preparations following limited cell disruption and subsequent fusion.

Different methodologies can be used to promote the interaction between infectious agent and donor membrane preparations, and/ or between the resulting fused membrane preparation and the target cells. For instance, it is known to use polyethylene glycol (PEG) to create an immortal antibody-producing cell line by the fusion of an intact antibody-producing B cell with a melanoma cell type. PEG can also be used to fuse crude cell lysates/enriched membrane preparations with similar material from the infective source. The same applies to ultimate fusion of the resulting fused membrane preparation and the target cells. Other polymers that compete for water, such as dextrans and poly(vinylpyrrolidone) (PVP), also increase intermolecular interactions between different membrane species. Other lipids such as DNA transfection agents, O-alkylphosphatidylcholines, may also be used to promote fusion of membranes.

Another means for promoting the interaction between infectious agent and donor membranes (also between the resulting fused membranes and the target cells) is buy altering the lipid content present in the lipid bilayers themselves prior to cell lysis and purification by adding chemicals to the cell media. Thus, in one embodiment, infectious agent, donor membranes and/ or target cells are treated to remove cell surface components that might interfere with the infection process, or in the case of infectious agent and donor membranes to enrich for fractions such as cellular membrane sub-fractions, lipid rafts or endosomal membranes that potentially enhance the infection process. By way of example, it is known that glycerol monoleate, oleic acid and arachidonic acid are all capable of destabilising the bilayer and thus promote membrane fusion (McIntosh, 1999). Furthermore, the addition of a ceramide synthase inhibitor, such as fumonisin B1, reduces the amount of sphingolipids present in lipid rafts. This has been shown to lead to increased levels of PrP^{sc} in an infected cell (Naslavsky, 1999).

In one embodiment, sonication may be used to destabilise the membranes, thereby assisting interaction between infectious agent and the donor membranes, and/ or between the fused membranes (ie. donor membranes with integrated infectious agent) and the target cells.

Thus, the extent of membrane fusion may be controlled using a combination of physical and chemical means.

In one embodiment, the target cell line used for the infections may match the animal species and/ or cell-type in which the infectious agent (ie. the TSE agent) has been detected. By way of example, the mouse neuroblastoma cell line N2a may be infected with mouse-passaged BSE-301V or vCJD. Also by way of example, human neuroblastoma cell lines such as Kelly, NB69 and SK-N-SH may be infected with human vCJD material Primary or immortalised non-neuronal cells, of bovine or mouse origin, derived from the dorsal root ganglia can be used as the target for infection with BSE or mouse passaged BSE material respectively. In addition, non-neuronal human cells, such as the Caco-2 cell line derived from the gut, can be used as the target for infection with vCJD material.

In a preferred embodiment, the target cell line and the cell line in which the infectious agent has been detected are both bovine. In a more preferred embodiment, the target cell line and the cell line in which the infectious agent has been detected are both human.

In an alternative embodiment, the target cell line used for the infections need not match the animal species/ cell-type in which the TSE agent has been detected, resulting in a mixed species infection. By way of example, mouse N2a target cell lines may be infected with human vCJD or non-variant CJD material, such as the familial or sporadic forms of the disease. The rat PC12 cell line of, neuronal phenotype may be infected with human vCJD or non-variant CJD, or BSE material. The human non-neuroblastoma cell line, Caco-2, may be infected with non-human material, such as mouse passaged BSE or scrapie.

Thus, in one embodiment, a mouse cell line may be infected with guinea pig infectious agent, such as guinea pig scrapie.

Corresponding selection criteria may be applied to the choice of intermediate (ie. donor) membrane employed. This selection may be based on a match with the donor membrane and/ or with the animal species/ cell-type in which the infectious agent has been detected, for example, as described above. In a preferred embodiment, the selection is based on a match between the donor membrane and the target cell. By way of example, mouse N2a donor membranes may be combined with mouse passaged BSE to infect a murine cell line, such as N2a. Human donor membranes derived from Kelly cells may be combined with vCJD or non-variant CJD material to infect a human cell line. The match is preferably performed at the species level, though may also (or separately) be performed at the cell-type level.

In an alternative embodiment, however, match criteria need not be applied between the donor membrane and/ or the animal species/ cell-type in which the infectious agent has been detected. By way of example, murine donor membranes from N2a cells may be combined with human material, such as vCJD or non- vCJD material for infecting human derived cell lines. Conversely, human membranes from NB69 cells may be mixed with mouse passaged BSE material for infecting murine derived cells.

In a preferred embodiment, a matching animal species and cell-type combination is employed. For example, chronic wasting disease (CWD) infectious material is combined with donor membranes derived from a human or mouse neuroblastoma cell line. This fusion is then used to inoculate the same cell type that provided the donor membranes, thus creating a CWD infected mouse or human cell line.

To increase the variety of mixed species/ material that may be generated, the target cell type to be infected may be modified to more closely match the species and/ or cell-type from which the infectious material has been derived. For instance, using an N2a (ie. mouse) target cell type to be infected with vCJD infectious material as an example, the target cell line may be first modified using RNA interference (RNAi) to 'knock-out' the expression of the endogenous murine PrP^{c}. Using conventional recombinant expression technology, this 'knock-out cell line is then modified to express the human PrP^{c} gene (ie. a N2a^{hPrPc} cell). Intermediate (ie. donor) membranes are purified from this new cell type, mixed with vCJD infectious agent, and then used to infect the original N2a^{hPrPc} target cell line. A similar approach may be used with the familiar or sporadic forms of the disease.

The 'transgenic' donor membranes described above may also be used to facilitate infection of wild-type target cell lines. Using the N2a example, membranes are purified from the N2a^{hPrPc} cell type, mixed with vCJD and then used to infect an unmodified murine neuroblastoma cell type such as the GT1 strain.

Other combinations include modifying murine or human neuroblastoma cell lines to express a cervid PrP^{c} to create a CWD *in vitro* assay.

More complex 'transgenic' donor membranes may be employed by fusing membranes from the target cell with membranes derived from a non-infected cell line of the same species as the infectious agent. Again, using the N2a example, N2a purified membranes are fused with purified membranes derived from a human neuroblastoma cell line, such as Kelly's, to create a complex 'transgenic' membrane preparation. This is fused in a second step with the vCJD agent and used to infect the N2a cell line. Likewise, a similar process may be used to infect a human neuroblastoma cell line, such as NB69's, with mouse passaged BSE or CWD.

According to a second aspect of the present invention, there is provided a method for detecting the presence of a TSE agent in a cell sample, comprising:
iii) contacting said target cell with a membrane preparation, wherein the membrane preparation comprises the TSE agent and a donor membrane;
iv) infecting the target cell with the TSE agent; and
v) detecting the presence of the TSE agent in the target cell.

All of the embodiments described above for the first aspect of the present invention apply equally to the second aspect of the present invention.

By use of this aspect of the invention, a TSE agent (present in an infected sample) may be readily passaged, thereby providing a simple and speedy means for propagating said TSE agent to provide an amount thereof suitable for detection and optionally strain typing (eg. by antibody detection or DNA sequencing).

One area of application for cell lines produced by the method of the present invention lies in the ability to check potentially infected samples, such as donated blood (eg. leukocytes), for the presence of infectious prion agents. In this regard, infectious prion agents identified in blood are associated with different fractions, with around 50% of the infectivity being extracellular and 50% associated with leukocytes. The leukocytes are routinely removed from donated blood in many countries, including the UK, to reduce the possibility of disease transmission, and this material is ideal for testing via a cellular infection model of the present invention.

However, whereas in vCJD and in scrapie there appear to be relatively large amounts of infectivity in blood, this is not the case with most sporadic and familial forms of CJD. The detection of these infectious agents depend on the identification of samples and/ or tissue that show infectivity and are reasonably accessible for sampling (eg. a range of peripheral tissues or cerebrospinal fluid). The rationale for the detection of prion agents in tissue samples is similar to that described for blood (Head, 2004).

Another potential risk of cross infection comes from the use of animal products for the production of biological drugs derived from cell culture. In this regard, a cell line could be contaminated with endogenous TSE agent, and/ or infection could be introduced from animal-sourced materials (eg. foetal calf serum) used in the growth or culture of the cell line. The method of the present invention is particularly useful for detecting TSE agent in these situations, where the amount of infective material us likely to be well below the level of sensitivity offered by *in vitro* tests such as Western blotting.

According to a third aspect of the present invention, there is provided a method for detecting the presence of a TSE agent strain type present in a cell sample, comprising:
i) contacting the test TSE agent, in separate assays, with a plurality of donor membrane preparations, wherein each donor membrane preparation comprises a membrane from a different animal species;
ii) in each assay, mixing the TSE agent and the respective donor membrane to provide a plurality of TSE-donor membrane mixes;
iii) contacting a plurality of said TSE-donor membrane mixes, in separate assays, with a target cell; and
iv) detecting the presence of the TSE agent in the target cells;
wherein the animal species of the donor membrane component of the target cell(s) in which TSE agent is detected is indicative of the strain type of the TSE agent in the sample.

A variation of the same embodiment, comprising:
i) contacting the test TSE agent, in separate assays, with a plurality of donor membrane preparations;
ii) in each assay, mixing the TSE agent and the respective donor membrane to provide a plurality of TSE-donor membrane mixes;
iii) contacting a plurality of said TSE-donor membrane mixes, in separate assays, with a plurality of target cells, wherein each target cell is from a different animal species;
iv) detecting the presence of the TSE agent in the target cells;
wherein the animal species of target cell(s) in which TSE agent is detected is indicative of the strain type of the TSE agent in the sample.

All of the embodiments described in connection with the first aspect of the present invention apply equally to the third aspect of the present invention.

Given its speed and sensitivity, in one embodiment, the method of the present invention is also useful for strain typing. Differentiation between TSE strains is achieved by the use of multiple target cells (or multiple donor membranes) that differ in their susceptibility to the TSE agent in question. By way of example, the diagnosis of a BSE infection in sheep may be achieved by incubating sheep brain material with a range of target cell -lines in accordance with the presence invention. Accordingly, the infectious agent will only infect those cell lines that are susceptible to the agent. Alternatively, the cell lines may demonstrate a range of susceptibility to infection. Referring to the above BSE example, the pattern of infection will be different from that of, for example, scrapie or CJD, which will demonstrate a different selectivity for different cell lines.

To achieve this selectivity, the target cell lines may be derived from a number of different animal species. Alternatively, to achieve selectivity, the target cell lines may be differentiated using a range of conventional agents to modify cellular response. As a yet further alternative, the target cell lines may be treated with different conventional glycoprotein/ glycolipid modifying agents to alter the surface chemistry of the cell lines. Suitable techniques and reagents are conventional (Naslavsky, 1999).

As with the second aspect of the present invention, infection is preferably detected by antibody binding to PrP^{sc}, and by subsequent visualisation by way of, for example, Western blotting or ELISA.

According to a fourth aspect of the present invention, there is provided a method for assessing the efficacy of a decontamination technology against a TSE agent, said method comprising:
i) exposing the TSE agent to the decontamination technology;
ii) contacting a first target cell with a test membrane preparation, wherein the test membrane preparation comprises the exposed TSE agent and a donor membrane, and infecting the target cell with the exposed TSE agent;
iii) contacting a second target cell with a control membrane preparation, wherein the control membrane preparation comprises unexposed TSE agent and a donor membrane, and infecting the target cell with the unexposed TSE agent; and
iv) detecting the presence of the TSE agent in the first and second target cell;
wherein a reduction in TSE agent in the first cell as compared to the second cell indicates that the decontamination technology is effective.

All of the embodiments described in connection with the first aspect of the present invention apply equally to the fourth aspect of the present invention.

In one aspect, the first and second target cells are from the same animal species, preferably both from the group consisting of human, sheep, cow, mouse and deer.

In one aspect, the target cells are of the same cell type (eg. neuronal cells such as neuroblastoma cells, or fibroblast cells).

In one aspect, the donor membrane component of the test membrane preparation and the control membrane preparation are from the same animal species (preferably both from the group consisting of human, sheep, cow, mouse and deer).

Any decontamination technology may be assessed using this method, including decontamination processes and/ or decontamination chemicals.

Reference to an 'exposed TSE agent' means a TSE agent that has been exposed to the decontamination technology. Thus, an 'unexposed TSE agent' is a TSE agent that has not been exposed to the decontamination technology.

In one aspect, the TSE agent may be exposed to the decontamination technology in solution. Following exposure to the decontamination technology, the sample may require further treatment - for example centrifugation and/ or washing - in order to separate the treated agent from the decontamination technology, thereby preventing excessive cell death following incubation with the target cell.

Alternatively, the TSE agent may be exposed to the decontamination technology on a solid phase, for example a metal disc - thereby allowing an assessment to be made of decontamination technologies for use on surgical instruments. Thus, in one aspect, the fourth aspect of the invention further comprises the initial step of drying down the TSE agent onto a solid phase, prior to exposing to the decontamination technology (eg. a cleaning or disinfection technology). The donor membranes may be incubated with the treated solid phases, enabling fusion and conversion events to occur.

As with the second and third aspects of the present invention, infection is preferably detected by antibody binding to PrP^{sc}, and by subsequent visualisation by way of, for example, Western blotting or ELISA.

The effectiveness of the decontamination technology may be assessed as a log reduction of infectivity by comparing the number of cells infected for the test fusions with the number of cells infected from the control fusions: Thus, the fourth aspect of the invention allows direct comparisons to be made of the effectiveness of different decontamination technologies.

The present invention is now described with reference to the Figures, in which:
Figure 1A illustrates cell morphologies of N2a cells following challenge with BSE-301V;
Figure 1B illustrates immunodetection of PrP^{C/Sc} in cell populations.
Figure 2 illustrates immunodetection of proteinase-K resistant PrP for cells challenged with BSE-301V.

In more detail, Figure 1A shows the distinctive cell morphologies of N2a cells following challenge with BSE-301V. In panels 1 and 2, the development of extended neurites can be seen. Large granulated cells with clear vacuolation are illustrated in panels 3-6. These granulated cells also showed clustering of normal N2a cells on their cell surface (panels 3 and 5). No equivalent morphologies were observed for N2a cells in culture at a comparative stage. Magnification x20 (panels 1 and 2), x80 (panels 3-6).

Figure 1 B illustrates ELISPOT showing immunodetection of PrP^{C/Sc} in cell populations. Panel A illustrates immunostaining of PrP^{c} for N2a cells in media alone (panel A1), following addition of normal brain homogenate (2), or microsomal membrane preparation (3). The PrP^{C} signal is removed following proteinase-K digestion (see panel 4). As a positive control; BSE-301V infectious brain homogenate was filtered and digested with Proteinase-K demonstrating the detection of PrP^{Sc} (panel 5). Panel B illustrates immunostaining of individual N2a cell clones after challenge with BSE-301V infectious brain homogenate and subsequent culture. Cell clones show different patterns of immunostaining, which remains after digestion with Proteinase-K (panels B1-6), thus confirming cell infectivity.

Figure 2 illustrates ELISPOT showing immunodetection of proteinase-K resistant PrP for cells challenged with BSE-301V. Cells challenged with microsomal membrane preparation alone (MMP control) or in combination with BSE-301V infectious mouse brain homogenate (from initial wells C10, C7, C2, F4, E5) were filtered through an ELISPOT plate. All samples, except for the MMP control, were treated with Proteinase-K (PK, 5µg/ml, 37°C 90mins) according to the protocol described in the Examples, below. The membrane was blocked, probed with 6H4 (Prionics) followed by anti-mouse alkaline phosphatase and colour developed with TMB. Proteinase-K treatment removed signal in the MMP control group, but signal remained in several clones derived from wells challenged with the MMP-iMBH preparation (notably C7 clone 3, 7 and 8, C2 clone 3, F4 clone 1 and E5 clone 2, 4, and 5).

### EXAMPLES

### Example 1 - Infection of a neuroblastoma cell line by prion agents.

Generation of membrane preparations from infectious brain material.

Infectious membrane fractions were isolated by an initial disruption of the intact organelle followed by differential centrifugation to purify the membranes. A crude 20% (w/v) homogenate was first generated by homogenising the brains in Phosphate Buffered Saline (PBS) using a Stomacher. The resulting crude homogenate was further sheared using a hand-held safety pestle and mortar comprising a PTFE plunger and glass vessel. The resulting fine suspension was subjected to 3 centrifugation steps: Step 1, 1000g, 5 mins., to remove large particulate material; step 2, 10000g, 7 mins., to remove intact organelles such as nuclei and mitochondria; step 3, 100000g, 60 mins., to pellet the microsomal membranes. The microsomal membranes were re-suspended in PBS to 20% (w/v) and stored at -70°C until required. Alternatively, if a more crude microsomal membrane preparation was required, the final centrifugation step consisted of 26000g, 120 mins.

### Culture of neuroblastoma cell lines, and preparation for infection.

Mouse neuroblastoma cell lines, such as N2a or GT1, or human neuroblastoma cell lines, such as Kelly, NB69 or SK-N-SH were cultured using standard cell culture techniques. The media used was based on either RPMI, DMEM or MEM:HAMS F12 with 10-15% Foetal Calf Serum (FCS) added in the presence or absence of 2mM Glutamine and 1% non-essential amino acids. In addition, between 1-5% glucose was added to increase the level of PrP^{c} expression and thereby assist the process of infection. When preparing the cells for infection they were split using an appropriate ratio to achieve a cell density of approximately 1x104 cells per well in a 24 well plate. Two days after splitting the cells were ready to be infected.

### Mixing and incubation of membrane cell preparations.

This method results in a new presentation of the infectious agent associated with membrane associated co-factors from the target cell to aid infection in the cellular model.

The target cell line to be infected was used to prepare a microsomal membrane fraction, as follows. Target cells were grown under the same conditions as described above. Approximately 1x10⁷ cells were harvested, washed with PBS and lysed. The cells were briefly re-suspended in buffer containing 10mM HEPES, pH 7.5 + 1 mM EDTA, protease inhibitor cocktail (Complete, Roche Diagnostics GmBH) and sheared by passing through a 23G needle 5 times. An equal volume of the above lysis buffer containing 500mM sucrose was added to maintain the integrity of the nuclei. The suspension was syringed again and centrifuged at 6000g for 10 mins to pellet large particulate matter. The supernatant was centrifuged at 15500g for 4 hours at 4°C. The resulting supernatant was discarded and the pellet, containing a crude microsomal membrane preparation, was re-suspended in PBS.

Fusion of the crude microsomes from the target cell with infectious material in the form of either crude homogenate or an enriched membrane preparation was achieved by combining the components at a ratio of 100 µg target microsomes to 1500 µg infectious material (ie. 1:15 ratio) and syringing through a safety needle a total of 3 times. Half of the fused material was then applied to the target cells per well in a 24 well plate.

### Culture and analysis of infected cell lines.

Two days after exposure to the infectious agent, the media was replaced and the growth rate of the cells monitored. The growth rate soon after infection tended to reduce and therefore the cells did not require splitting for 7-10 days. In this situation fresh media was added on top of the cells to maintain cell viability. The cells were split twice when confluent and then analysed for infectivity (see below). Infected cells were maintained in standard cell culture media as described previously with glucose added and split as required.

The first indication that the cells have been infected is that there is considerable cell death, with the remaining cells having a distinct morphology (figure 1). The infected cells have a large degree of vacuolation, which could be lysosomal in nature as the cells may be attempting to degrade the infective material. The surviving cells produced neurites extending from the main cellular body - indicating that differentiation events were taking place.

Confirmation of infectivity was performed using a cell blot technique based on the method described by Klohn *et al.* (2003). After splitting, approximately 5000 cells were placed into one well of a 96-well filtration plate (Immobilon-P, Millipore) and washed with PBS via suction and allowed to dry for 1 hour.

For assessing the presence of PrP^{sc} the dried cells were treated with 5µg/ml Proteinase K in cell lysis buffer (10mM Tris, 150mM NaCl pH 7.2, 0.5% Deoxycholate, 0.5% Triton) for 90 mins at 37°C. The membrane was then washed twice with PBS and incubated for 25mins at room temperature with the protease inhibitor APMSF (5mM in PBS). The protein on the membrane was denatured in Urea buffer (6M Urea in 10mM Tris pH 8.0) for 10 minutes at room temperature. The membrane was washed 4 times with PBS, blocked for 45 minutes at room temperature with 5% skimmed milk in PBS and washed once with PBS. The primary antibody 6H4 was added at 1:1000 dilution in PBST containing 1% skimmed milk and the membrane incubated for 1 hour at 37°C. The membrane was washed 4 times with PBST and then incubated with anti-mouse alkaline phosphatase (1/1000 in PBST) for 2 hours at 37°C. After washing the membrane 4 times with PBST the bound antibodies were detected by the addition of BCPT/NBT reagents. Following colour development the plate was washed with water and imaged using a digital camera.

Figure 2 illustrates identification of infected cells using the cell blot method. The pattern of spots on the membrane relates to distinct infected cell colonies and provides an estimation of the level of infectivity present in the original test sample.

The utilisation of ELISpot counting equipment considerably increases the number of samples that can be screened using this method and has a sensitivity approaching that of the mouse bioassay.

Further confirmation of infectivity can be achieved by using a variety of different antibody based detection systems. Western blotting was performed using phosphotungstic acid (PTA) to enrich for PrP^{sc} in cell lysates (Safar *et al*., 1998). PTA selectively precipitates PrP^{sc} whilst leaving the non-infectious form, PrP^{c} in solution. Between 5000-20000 cells were lysed in 300ml of 10mM Tris/HCl, pH 7.4 + 1% NP40, 1% Triton X-100, 4% Sarkosyl: PK was added to 25mg/ml and incubated for 1 hour at 37°C. The digestion was stopped by addition of Pefabloc (Roche Diagnostics GmbH) to 1 mM, and PTA added to 0.3% in the presence of 170 mM MgCl2, pH 7.4. After 16h at 37°C the precipitated PrPsc was pelleted by centrifugation and analysed by Western blotting using the prion specific antibody, 6H4, as described above. The pattern of banding on the blot can also aid identification of the strain type (see Example 8). To detect Proteinase sensitive PrP^{sc} isoforms, the PTA methodology was incorporated into the conformation-dependent immunoassay (CDI, Safar *et al.,* 1998). Cell extracts were prepared in duplicate as described above with one aliquot denatured with GdnHCI. The europium-labelled anti-prion antibody, 3F4, is only able to recognise its epitope in PrP^{sc} after denaturation and therefore the ratio of binding of the antibody between native and denatured forms of the sample enables the amount of PrP^{sc} present to be determined via fluorometeric means.

### Example 2 - Methods for promoting the interaction and fusion of model membranes

To promote fusion of membranes from different cell types, the two components were sheared through a fine needle (see Example 1).

To characterise the vesicles, electron microscopy (EM) was utilised to determine the size range of the particles formed. Vesicles that are too large (>250nm) may not be as effectively endocytosed by the target cell and therefore affect infectivity. However, the size range of the particles produced can be controlled by varying the energy input into the system and using different concentrations of chemicals. In addition, EM advantageously enables estimation of the percentage of membranes derived from the rough endoplasmic reticulum (rER) in the microsomal membrane preparation. By using a series of marker enzymes specific for membranes derived from the major cell organelles, the precise make up of the membrane preparation may be further characterised. For example, determination of 5'-nucleosidase, activity indicates the presence of plasma membrane fragments, and determination of b-D-galactosidase activity indicates the presence of lysosomal derived material.

### Example 3 - Isolation of enriched plasma membrane and endosomal membranes.

Different types of donor membrane and / or infectious agent can be used to achieve infection or to varying the selectivity of the assay. To prepare a more enriched plasma preparation the homogenates derived from tissue or cells are subjected to a low speed spin as for preparing a crude microsomal membrane preparation. The pellet from this step is re-suspended in 71% sucrose, transferred to centrifuge tubes and overlaid with 53% and 42% sucrose and centrifuged at 100000g for 1 h. Highly pure plasma membranes band at and just above the 42% - 53%. Using different concentrations of sucrose, purification of membranes associated with endocytosis, such as lysosomes or endosomes can also be achieved. Combining the above sucrose gradient centrifugation technique with Triton X-100 phase separation allows a further level of purification to be achieved resulting in the isolation of particular microdomains, 'lipid rafts', from within the membranes.

### Example 4 - Screening of donated blood for the presence of prion infectious agents

Leukocytes are routinely removed from a donated blood, and a variety of filtration devices are commercially available and will be known to those familiar with the field. Following blood donation and leukoreduction the filter unit is disassembled and the filter washed to isolate the leukocyte fraction.

The leukocytes may be treated at this point to facilitate the infection process. By way of example, the leukocytes may be treated to isolate specific membrane fractions, to remove GPI-anchored infectious prion from the surface of the cell, or to remove glycosyl groups from cellular glycoproteins via one of the methods described above.

The target cell line is preferably a human neuroblastoma cell line, such as Kelly or NB69 cells (Schwab, 1983), but other human cell lines may also be suitable to support the infection process.

The infectious cell fractions (prepared as described above) are sheared together with a donor cell membrane preparation by repeated passage through a 23G needle: Viable target cells are then exposed to the fusion, followed by subsequent plating onto tissue culture plates. The cells are cultured for a period of 7-14 days, or possibly longer if required for infectivity to be manifested, and the cell pellets and/ or supernatant are collected by ELISpot onto a nitrocellulose or PVDF membrane.

The ELISpot is probed with an anti-prion antibody (eg. 6H4 Prionics), which has an epitope that is resistant to proteinase K digestion of the membrane-filtered preparations. This is taken to identify the presence of protease-resistant mis-folded PrP^{Sc}, which is believed to be indicative of the disease. Alternatively, the filter is probed with an antibody that is specific for the mis-folded form and does not recognise the normal cellular form, PrP^{c}. Preferably, the antibody is specific for the form of the infectious prion that is present in blood, thereby providing additional specificity for the detection method. Positive samples are those identified as having prion-immunoreactive material when compared to the negative control of uninfected cells and normal leukocytes.

The units of blood are quarantined until the results of the tests are known and, if negative for prion infectivity, the units are subsequently released for use.

### Example 5 - Detection of infectious prion in other samples

### Sporadic CJD

CSF samples are taken from individuals suspected of having sporadic CJD. The sample is centrifuged to isolate intact cells from the CSF that are likely to include astrocytes, glial and potentially some neuronal cells (although the presence of neuronal cells is not essential for the success of the assay). The cell pellet is mixed with a donor membrane preparation, for example, derived from the target cell line, such as human neuroblastoma cells (such as SK-N-SH cells) and the membranes fused by the use of PEG or similar treatments. Viable target cells are exposed to the fused membrane preparation and are then plated onto tissue culture plates and the samples incubated for the required period of time. Infection is detected by ELISpot with the membrane probed with suitable anti-prion antibodies either directly or following digestion with Proteinase K.

Optionally, the approaches described in Examples 1-3 may be used to optimise and/ or enhance the uptake of infectious material into the cell line.

### Scrapie

A second example relates to the detection of scrapie from cells isolated from a buccal swab from a suspect sheep. These cells are collected by centrifugation as above and mixed with a membrane preparation derived from a target cell, such as the N2a mouse neuroblastoma cell line. Optionally, a transgenic cell line expressing an ovine PrP in the N2a cell line, in which the native mouse prp gene has been knocked out by conventional techniques, may be used as the target cell line. The buccal cells are incubated with a donor membrane preparation, for example, derived from the target cell, with the membranes brought together by either shearing or membrane fusion (as described above). Viable target cells are then incubated with the fused membranes and are plated and assayed as previously described.

### Example 6 - Use of the cell infection model to test pharmaceutical cell culture lines for prion infection.

The target cell line of interest is grown under standard conditions as required. Cells are taken from actively growing cultures or from cultures that have been differentiated to halt cell cycle progression. The cells are collected, pelleted by centrifugation and the cell pellet mixed with a donor membrane preparation (eg. derived from the target cells prepared using one of the methods described above). Shearing or sonication based fusion methods help to ensure that the pelleted cells are lysed during this process and help to ensure efficient integration of the two-component system. The resulting membrane fusion is then used to inoculate target cell preparations, which are plated and grown for sufficient time to allow the infection of the target cell, and then grown on to provide sufficient material for assay. The cells may then be assessed for infection by ELISpot or Western blot as appropriate.

### Example 7 - Use of an infected cell line for testing anti-prion drug candidates

Infected target cell lines are generated by one of the methods described above. Once established, the cell lines can be used to test agents proposed to have some form of anti-prion activity. For example, a monoclonal antibody that binds to PrP^{Sc} is incubated with the cell line at a variety of concentrations and over varying time courses. The cell line is assessed for the continuing production of PrP^{Sc} following antibody treatment and this is compared to untreated cell controls and cells treated with an antibody of the same isotype but not directed to PrP. Either ELISpot or Western blot with appropriate antibodies is used to assess the effects of the antibody on prion propagation. Potentially interesting candidates are identified as those that reduce the levels of PrP^{Sc} produced by the cell and that do so at concentrations that are consistent with therapeutic use.

Similar methods apply to the screening of drug candidates, for the use of "gene targeting" strategies aimed at preventing PrP^{Sc} accumulation, and for other therapeutic methods.

### Example 8 - Use of cellular infection model for TSE strain typing

A panel of different target cell lines are set up using a variety of different techniques.

Initially, neuroblastoma or other target cell models from a range of different species and/or PrP genotypes are identified. This might, for example, include human neuroblastoma cell lines (eg. Kelly, NB69, SK-N-SH); mouse/rat neuroblastomas (N2a, PC12); transgenic cell lines with altered prp genes (eg. N2a with ovine, bovine or human prp gene); and immortalised cell lines from different regions of the brain (eg. hippocampus, substantia nigra, medulla, thalamus and others).

These cell lines may be treated additionally with chemical reagents that alter the glycosylation pattern of certain proteins and/or the surface properties of the cell line (eg. reagents to modify cholesterol synthesis, ganglioside deficient N2a cell line, glycosyltransferase-negative cell lines (eg. alpha(1,2)fucosyltransferase), glycohydrolase-negative cell lines or chemical reagents that achieve similar effects), or which alter intracellular trafficking of membrane bound vesicles (eg. botulinum neurotoxins, brefeldin A, bafilomycin). The precise panel of cell lines to be used for the study may depend on the infectious agent and its origins.

To assess the strain type, the sample (eg. brain or white blood cells) is first mixed with a donor membrane preparation purified from a range of different target cells according to the protocols identified above. The cell membranes and the infectious agent to be tested are mixed together either with or without shearing, as appropriate, and applied to the corresponding target cell line to be infected, and the cells plated on to tissue culture plates. The cell lines are grown for a minimum of 7 days and the cell infection assessed by ELISpot and/or Western blot. The definition of strain type is dependent on the range of cell lines infected and the correlation of this information with previous studies with TSE strains of defined origin (Bruce, 2002).

### Example 9 - Use of infected cell lines to assess the efficacy of decontamination technologies against TSE agents in solution

A panel of cell lines are set up as described above (Example 8), together with their respective donor membranes.

To demonstrate the effectiveness of a decontamination technology against a TSE agent in solution, the agent is first exposed to the decontamination process or chemical for the specified length of time. Following exposure, the sample may optionally require centrifugation and/ or washing to separate the treated agent from the decontamination process or chemical, to prevent excessive cell death.

The donor membranes from the respective cell lines are fused with the treated agent by one of the methods described above, and the cells are plated onto tissue culture plates. Similarly, control fusions are set up, consisting of the untreated TSE agent with the donor membranes. Following a suitable length of time, the extent of infection is determined using the ELISpot method.

The effectiveness of the decontamination technology can be expressed as a log reduction of infectivity by comparing the number of cells infected form the test fusions with those of the control fusions.

### Example 10 - Use of infected cell lines to assess the efficacy of decontamination technologies against TSE agents on surgical instruments

The infectivity assay can also be used to evaluate decontamination technologies for use on surgical instruments.

The infectious agent is dried down onto a suitable solid phase token (eg. a metal disc) and exposed to the cleaning or disinfection technology. Donor membranes are incubated with the treated tokens, using methods described previously, to enable fusion and conversion events to occur, followed by incubation with the target cells. Similarly, control fusions are set up, consisting of the untreated TSE agent with the donor membranes. Cells are plated out and the number infected is determined by ELISpot.

Again, a log reduction in infectivity can be determined by comparison to the untreated infectious control group, enabling direct comparisons to be made on the effectiveness of different decontamination technologies.

### References

D.O.V. Alonso, S.J. DeArmond, F.E. Cohen and V. Daggett, Mapping the early steps in the pH-induced conformational conversion of the prion protein, Proc. Natl. Acad. Sci. USA 98 (2001) 2985-2989.
Anon. Update: Creutzfeldt-Jakob disease in a patient receiving a cadaveric dura mater graft. MMWR Morb Mortal Wkly Rep. 36 (1987) 324-325.
C. Bernoulli, J. Siegfried, G. Baumgartner, F. Regli, T. Rabinowicz, D.C, Gajdusek, and C.J. Jr.Gibbs. Danger of accidental person-to-person transmission of Creutzfeldt-Jakob disease by surgery. Lancet. 1 (1977) 478-479.
C. Bordier. Phase separation of integral membrane proteins in Triton X-114 solution. J Biol Chem. (1981);256 (4) 1604-1607
D. C. Bolton, M. P. McKinley and S. B. Prusiner, Identification of a protein that purifies with the scrapie prion, Science 218 (1982) 1309-1311.
F.J. Brooke, A. Boyd, G.M. Klug, C.L. Masters and.S.J. Collins. Lyodura use and the risk of iatrogenic Creutzfeldt-Jakob disease in Australia Med J Aust. 180 (2004) 177-181.
P. Brown, M. Preece, J.P. Brandel, T. Sato, L. McShane, I. Zerr, A. Fletcher, R.G. Will, M. Pocchiari, N.R. Cashman, J.H.. d'Aignaux, L. Cervenakova, J. Fradkin, L.B. Schonberger and S.J. Collins. Iatrogenic Creutzfeldt-Jakob disease at the millennium. Neurology. 55 (2000) 1075-1081.
M. Bruce, R.G. Will, J.W. Ironside, I. McConnell, D. Drummond, A. Suttie, L. McCardle, A. Chree, J. Hope, C. Birkett, S. Cousens, H. Fraser, and C.J. Bostock, Transmissions to mice indicate that 'new variant' CJD is caused by the BSE agent, Nature 389 (1997) 498-501.
M.E. Bruce, A. Boyle, S. Cousens, I. McConnell, J. Foster, W. Goldmann and H. Fraser, J Gen Virol 83 (2002) 695-704
B. Caughey, G.J. Raymond, D.A. Kocisko and P.T. Lansbury, Cell-free formation of protease-resistant prion protein, J. Virol. 71 (1997) 4107-4110.
HJ. Cho, Inactivation of the scrapie agent by pronase, Can. J. Comp. Med. 47 (1983) 494-496.
S. Collins, M.G. Law, A. Fletcher, A. Boyd, J. Kaldor, and C.L. Masters. Surgical treatment and risk of sporadic Creutzfeldt-Jakob disease: a case-control study. Lancet. 353(1999) 693-697.
E.A. Croes, G. Roks, G.H. Jansen, P.C. Gnijssen, and C.M. van Duijn. Creutzfeldt-Jakob disease 38 years after diagnostic use of human growth hormone Neurol Neurosurg Psychiatry;72 (2002) 792-793.
B.S. De Silva, K.L. Egodage and G.S. Wilson. Purified protein derivative (PPD) as an immunogen carrier elicits high antigen specificity to happens. Bioconjug Chem. 10 (1999) 496-501.
H. Diringer, H. Gelderblom, H. Hilmert, M. Ozel, C. Edelbluth and R.H. Kimberlin, Scrapie infectivity, fibrils and low molecular weight protein, Nature 306 (1983) 476-478.
G. Fichet, E. Comoy, C. Duval, K. Antloga, C. Dehen, A. Charbonnier, G. McDonnell, P. Brown, C.I. Lasmezas and J.P. Deslys. Novel methods for disinfection of prion-contaminated medical devices. Lancet. 364 (2004) 521-6.
M. Glatzel, E. Abela, M. Maissen, and A. Aguzzi. Extraneural pathologic prion protein in sporadic Creutzfeldt-Jakob disease. N Engl J Med. 349 (2003) 1812-1820.
A.H. Grobben, P.J. Steel, R.A. Somerville and D.M. Taylor, Inactivation of the bovine spongiform encephalopathy (BSE) agent by the acid and alkaline processes for manufacturing of bone gelatine, Biotechnol. Appl. Biochem. 39 (2004) 329-338.
D.L. Ritchie, M.W. Head, J.W. Ironside, Advances in the detection of prion protein in peripheral tissues of variant Creutzfeldt-Jakob disease patients using paraffin-embedded tissue blotting. Neuropathology & Applied Neurobiology 30 (2004), 360-368.
C. Herzog, N. Sales, N. Etchegaray, A. Charbonnier, S. Freire, D. Dormont, J.P. Deslys and C.I. Lasmezas. Tissue distribution of bovine spongiform encephalopathy agent in primates after intravenous or oral infection. Lancet. 363(2004) 422-428.
A.F. Hill, M. Desbruslais, S. Joiner, K.C. Sidle, I. Gowland, J. Collinge, L.J. Doey, and P. Lantos. The same prion strain causes vCJD and BSE. Nature. 389 (1997) 448-450.
A.F. Hill and J. Collinge. Subclinical prion infection. Trends Microbiol. 11 (2003) 578-584.
D.A. Hilton, A.C. Ghani, L. Conyers, P. Edwards, L. McCardle, M. Penney, D. Ritchie, and J.W. Ironside. Accumulation of prion protein in tonsil and appendix: review of tissue samples. BMJ. 325 (2002) 633-634.
S. Joiner, J. Linehan, S. Brandner, J.D. Wadsworth and J. Collinge. Irregular presence of abnormal prion protein in appendix in variant Creutzfeldt-Jakob disease. J. Neurol. Neurosurg. Psychiatry 73 (2002) 597-598.
P.C. Klohn, and L. Stoltze, et al. (2003). "A quantitative, highly sensitive cell-based infectivity assay for mouse scrapie prions." Proc Natl Acad Sci U S A 100(20): 11666-71.
D. A. Kocisko, J. H. Come, S. A. Priola, B. Chesebro, P.T. Lansbury and B. Caughey, Scrapie infectivity correlates with converting activity, protease resistance, and aggregation of scrapie-associated prion protein in guanidine denaturation studies, Nature 370 (1994) 471-474.
J.P. Langeveld, J.-J. Wang, D.F.M. Van de Wiel, G.C. Shih, G.J. Garssen, A. Bossers and J.C.H. Shih, Enzymatic degradation of prion protein in brain stem from infected cattle and sheep, J. Inf. Dis. 188 (2003) 1782-1789.
C.A. Llewelyn, P.E. Hewitt, R.S. Knight, K. Amar, S. Cousens, J. Mackenzie, and R.G. Will. Possible transmission of variant Creutzfeldt-Jakob disease by blood transfusion. Lancet. 363 (2004) 417-221.
N. Naslavsky, H. Shmeeda, G. Friedlander, A. Yanai, A.H. Futerman, Y. Barenholz and A. Taraboulos, J. Biol. Chem. 274 (1999) 20763-20771
A.H. Peden, M.W. Head, D.L. Ritchie, J.E. Bell, and J.W. Ironside. Preclinical vCJD after blood transfusion in a PRNP codon 129 heterozygous patient. Lancet. 364 (2004) 527-529.
SB. Prusiner, Novel proteinaceous infectious particles cause scrapie, Science 216 (1982) 136-144.
R. Rubenstein, R.I. Carp, W. Ju, C. Scalici, M. Papini, A. Rubenstein and R. Kascsak, Concentration and distribution of infectivity and PrPSc following partial denaturation of a mouse-adapted and a hamster-adapted scrapie strain, Arch. Virol. 139 (1994) 301-311.
Safar, J., H. Wille, et al. (1998). "Eight prion strains have PrP(Sc) molecules with different conformations." Nat Med 4(10): 1157-65.
Safar, J., M. Ceroni, et al. (1990). "Subcellular distribution and physicochemical properties of scrapie-associated precursor protein and relationship with scrapie agent." Neurology 40(3 Pt 1): 503-8.
Schwab, M., et al. (1983). "Amplified DNA with limited homology to myc cellular oncogene is shared by human neuroblastoma cell lines and a neuroblastoma tumour. Nature 305 (5931): 245-248.
A.J. Swerdlow, C.D. Higgins, P. Adlard, M.E. Jones, and M.A. Preece. Creutzfeldt-Jakob disease in United Kingdom patients treated with human pituitary growth hormone. Neurology. 61(2003) 783-791.
D.M. Taylor, H. Fraser, I. McConnell, D.A. Brown, D.L. Brown, K.A. Lamza and G.R. Smith, Decontamination studies with the agents of bovine spongiform encephalopathy and scrapie, Arch. Virol. 139 (1994) 313-326.
D.M. Taylor. Inactivation of transmissible degenerative encephalopathy agents: A review. Vet J. 159 (2000) 10-17.
D.M. Taylor, Transmissible degenerative encephalopathies. Inactivation of the causal agents, in: Principles and practice of disinfection, preservation and sterilisation. A.D. Russell, W.B. Hugo and G.A.J. Ayliffe (Eds.) Blackwell Scientific Publications, Oxford (1999) pp 222-236.
D.M. Taylor, Resistance of transmissible spongiform encephalopathy agents to decontamination, Contrib. Microbiol. 7 (2001) 58-67.
D.M. Taylor, K. Fernie, P.J. Steele, I. McConnell and R.A. Somerville, Thermostability of mouse-passaged BSE and scrapie is independent of host PrP genotype: implications for the nature of the causal agents, J. Gen. Virol. 83 (2002) 3199-3294.
D.M. Taylor. Resistance of transmissible Spongiform encephalopathy agents to decontamination. Contrib Microbiol. 11 (2004) 136-145.
H.J. Ward, D. Everington, E.A. Croes, A. Alperovitch, N. Delasnerie-Laupretre, I. Zerr, S. Poser, and C.M. van Duijn. Sporadic Creutzfeldt-Jakob disease and surgery: a case-control study using community controls. Neurology. 59 (2002) 543-548.
G.A. Wells, Pathology of nonhuman spongiform encephalopathies: variations and their implications for pathogenesis, Dev. Biol. Stand. 80 (1993) 61-69.
R.G. Will, Epidemiology of Creutzfeldt-Jacob disease, Br. Med. Bull. 49 (1993) 960-970.
J H. Wille, G.-F. Zhang, M.A. Baldwin, F.E. Cohen and S.B. Prusiner, Separation of scrapie prion infectivity from PrP amyloid polymers, Mol. Biol. 259 (1996) 608-621.
DJ Weber and WA Rutala. Creutzfeldt-Jakob disease: recommendations for disinfection and sterilization. Clin Infect Dis. 32(2001) 1348-1356.
DJ Weber and WA Rutala. Managing the risk of nosocomial transmission of prion diseases. Curr Opin Infect Dis. 15 (2002) 421-425.
S. van der Werf; J.P. Briand, S. Plaue, J. Burckard, M. Girard, and M.H. Van Regenmortel. Ability of linear and cyclic peptides of neutralization antigenic site 1 of poliovirus type 1 to induce virus cross-reactive and neutralizing antibodies. Res Virol. 145 (1994) 349-359.
ZX Yan, L Stitz, P Heeg, E Pfaff, K Roth. Infectivity of prion protein bound to stainless steel wires: a model for testing decontamination procedures for transmissible spongiform encephalopathies. Infect Control Hosp Epidemiol. 25(2004) 280-283.
W.Q. Zou and N.R Cashman, Acidic pH and detergents enhance in vitro conversion of human brain PrPC to a PrPSc-like form, J. Biol. Chem. 277 (2002) 43942-43947.

## Claims

1. A method for infecting a target cell line with a TSE agent, comprising:
i) forming a membrane preparation containing a TSE agent from a first animal cell-type within a first membrane; and mixing said membrane preparation with a second membrane from a second animal cell-type; wherein the first and second animal cell-types are different;
ii) forming a contiguous membrane comprising said TSE agent and said second membrane;
iii) contacting said target cell line with said contiguous membrane;
and thereby infecting said target cell line with said TSE agent;
wherein the second membrane is from the same animal cell type as that of the target cell line.

2. A method for infecting a target cell line with a TSE agent, comprising:
i) forming a membrane preparation containing a TSE agent from a first animal species within a first membrane, and mixing said membrane preparation with a second membrane from a second animal species, wherein the first and second animal species are different;
ii) forming a contiguous membrane comprising said TSE agent and said second membrane;
iii) contacting said target cell line with said contiguous membrane;
and thereby infecting said target cell line with said TSE agent;
wherein the second membrane is from the same animal species as that of the target cell line.

3. A method according to Claim 1, wherein the TSE agent from the first animal cell-type and the second membrane are from different animal species.

4. A method according to Claim 2, wherein the TSE agent from the first animal species and the second membrane are from different animal cell-types.

5. A method according to any preceding claim, wherein the TSE is selected from the group consisting of CJD, vCJD, sporadic CJD, familial CJD, iatrogenic CJD, BSE, ovine BSE, and CWD.

6. A method according to any preceding claim, wherein the source of the TSE agent is selected from the group consisting of lymphatic tissues (eg. tonsil, appendix, rectal tonsil), neuronal tissues (eg. brain, central nervous system), and blood (eg. lymphocytes).

7. A method according to any preceding claim, wherein the target cell line is selected from the group consisting of neuronal cells (eg. neuroblastoma cells), and fibroblast cells.

8. A method according to any preceding claim, wherein the second membrane and the target cell line are from an animal species selected from the group consisting of human, sheep, cow, mouse and deer.

9. A method according to any preceding claim, wherein the second membrane and the target cell line are from a cell-type selected from the group consisting of neuroblastoma cells (eg. NB69, SK-N-SH, and Kelly).

10. A method according to any preceding claim, wherein the target cell line and the cell from which the TSE agent is obtained are from the same animal species, preferably from the group consisting of human, sheep, cow, mouse, and deer.

11. A method according to any preceding claim, wherein the target cell line and cell from which the TSE agent is obtained are the same cell-type, preferably from the group consisting of neuroblastoma cells (eg. NB69, SK-N-SH, and Kelly).

12. A method according to Claim 2, wherein the second membrane and the cell from which the TSE agent is obtained are from the same cell-type, preferably from the group consisting of neuroblastoma cells (eg. NB69, SK-N-SH, and Kelly).

13. A method according to any of Claims 1-4, wherein the TSE agent is selected from the group consisting of CJD, vCJD, sporadic CJD, familial CJD, iatrogenic CJD, and wherein the target cell line and second membrane are from a non-human species (eg. from mouse, rat, cow, sheep, deer).

14. A method according to any of Claims 1-4, wherein the TSE agent is BSE, and wherein the target cell line and second membrane are from a non-bovine species (eg. from human, mouse, rat, sheep or deer).

15. A method according to any of Claims 1-4, wherein the TSE agent is CWD, and wherein the target cell line and second membrane are from a non-deer species (eg. from human, mouse, rat, cow or sheep).

16. A method according to any of Claims 1-4, wherein the TSE agent is ovine BSE or scrapie, and wherein the target cell line and second membrane are from a non-ovine species (eg. from human, mouse, rat, cow or deer).

17. A method according to any previous claim, wherein the first or second membrane is a microsomal membrane.

## Patentansprüche

1. Ein Verfahren zum Infizieren einer Ziel-Zelllinie mit einem TSE-Erreger, welches umfasst:
i) Bilden einer Membranpräparation, welche einen TSE-Erreger von einem ersten Tier-Zelltyp innerhalb einer ersten Membran enthält; und Mischen der Membranpräparation mit einer zweiten Membran von einem zweiten Tier-Zelltyp; wobei die ersten und zweiten Tier-Zelltypen verschieden sind;
ii) Bilden einer zusammenhängenden Membran, welche den TSE-Erreger und die zweite Membran umfasst;
iii) Berühren der Ziel-Zelllinie mit der zusammenhängenden Membran;
und dabei Infizieren der Ziel-Zelllinie mit dem TSE-Erreger;
wobei die zweite Membran von dem gleichen Tier-Zelltyp wie die der Ziel-Zelllinie ist.

2. Ein Verfahren zum Infizieren einer Ziel-Zelllinie mit einem TSE-Erreger, welches umfasst:
i) Bilden einer Membranpräparation, welche einen TSE-Erreger von einer ersten Tiergattung innerhalb einer ersten Membran enthält, und Mischen der Membranpräparation mit einer zweiten Membran von einer zweiten Tiergattung, wobei die ersten und zweiten Tiergattungen verschieden sind;
ii) Bilden einer zusammenhängenden Membran, welche den TSE-Erreger und die zweite Membran umfasst;
iii) Berühren der Ziel-Zelle mit der zusammenhängenden Membran;
und **dadurch** Infizieren der Ziel-Zelllinie mit dem TSE-Erreger;
wobei die zweite Membran von der gleichen Tiergattung wie die der Ziel-Zelllinie ist.

3. Ein Verfahren gemäß Anspruch 1, wobei der TSE-Erreger von dem ersten Tier-Zelltyp und die zweite Membran von verschiedenen Tiergattungen sind.

4. Ein Verfahren gemäß Anspruch 2, wobei der TSE-Erreger von der ersten Tiergattung und die zweite Membran von verschiedenen Tier-Zelltypen sind.

5. Ein Verfahren gemäß einem vorhergehenden Anspruch, wobei der TSE-Erreger aus der Gruppe bestehend aus CJD, vCJD, sporadisches CJD, familiäres CJD, iatrogenes CJD, BSE, ovines BSE, und CWD ausgewählt wird.

6. Ein Verfahren gemäß einem vorhergehenden Anspruch, wobei die Quelle des TSE-Erregers aus der Gruppe bestehend aus lymphatischen Geweben (z.B. Mandel, Wurmfortsatz, Analtonsille), neuronalen Geweben (z.B. Gehirn, zentrales Nervensystem), und Blut (z.B. Lymphozyten) ausgewählt wird.

7. Ein Verfahren gemäß einem vorhergehenden Anspruch, wobei die Ziel-Zelllinie aus der Gruppe bestehend aus neuronalen Zellen (z.B. Neuroblastom-Zellen), und Fibroblast-Zellen ausgewählt wird.

8. Ein Verfahren gemäß einem vorhergehenden Anspruch, wobei die zweite Membran und die Ziel-Zelllinie von einer Tiergattung aus der Gruppe bestehend aus Mensch, Schaf, Kuh, Maus und Wild ausgewählt wird.

9. Ein Verfahren gemäß einem vorhergehenden Anspruch, wobei die zweite Membran und die Ziel-Zelllinie von einem Zelltyp aus der Gruppe bestehend aus Neuroblastom-Zellen (z.B. NB69, SK-N-SH, und Kelly) ausgewählt wird.

10. Ein Verfahren gemäß einem vorhergehenden Anspruch, wobei die Ziel-Zelllinie und die Zelle, von welcher der TSE-Erreger erhalten wird, von der gleichen Tiergattung, vorzugsweise aus der Gruppe bestehend aus Mensch, Schaf, Kuh, Maus und Wild, sind.

11. Ein Verfahren gemäß einem vorhergehenden Anspruch, wobei die Ziel-Zelllinie und die Zelle, von welcher der TSE-Erreger erhalten wird, von dem gleichen Zelltyp, vorzugsweise aus der Gruppe bestehend aus Neuroblastom-Zellen (z.B. NB69, SK-N-SH, und Kelly) sind.

12. Ein Verfahren gemäß Anspruch 2, wobei die zweite Membran und die Zelle, von welcher der TSE-Erreger erhalten wird, von dem gleichen Zelltyp, vorzugsweise aus der Gruppe bestehend aus Neuroblastom-Zellen (z.B. NB69, SK-N-SH, und Kelly) sind.

13. Ein Verfahren gemäß einem der Ansprüche 1-4, wobei der TSE-Erreger aus der Gruppe bestehend aus CJD, vCJD, sporadisches CJD, familiäres CJD, iatrogenes CJD ausgewählt wird, und wobei die Ziel-Zelllinie und die zweite Membran von einer nicht-menschlichen Gattung (z.B. von Maus, Ratte, Kuh, Schaf, Wild) sind.

14. Ein Verfahren gemäß einem der Ansprüche 1-4, wobei der TSE-Erreger BSE ist, und wobei die Ziel-Zelllinie und zweite Membran von einer nicht-bovinen Gattung (z.B. von Mensch, Maus, Ratte, Schaf oder Wild) sind.

15. Ein Verfahren gemäß einem der Ansprüche 1-4, wobei der TSE-Erreger CWD ist, und wobei die Ziel-Zelllinie und zweite Membran von einer Nicht-Wild Gattung (z.B. von Mensch, Maus, Ratte, Kuh oder Schaf) sind.

16. Ein Verfahren gemäß einem der Ansprüche 1-4, wobei der TSE-Erreger ovines BSE oder Scrapie ist, und wobei die Ziel-Zelllinie und zweite Membran von einer nichtovinen Gattung (z.B. von Mensch, Maus, Ratte, Kuh oder Wild) sind.

17. Ein Verfahren gemäß einem vorhergehenden Anspruch, wobei die erste oder zweite Membran eine Microsomal-Membran ist.

## Revendications

1. Procédé pour infecter une lignée cellulaire cible avec un agent EST (d'encephalopathie spongiforme transmissible) comprenant les étapes consistant à :
i) former une préparation membranaire renfermant un agent EST provenant d'un premier type de cellule animale dans une première membrane, et mélanger cette préparation membranaire avec une seconde membrane provenant d'un second type de cellule animale, le premier et le second type de cellule animale étant différents,
ii) former une membrane contiguë comprenant l'agent EST et la seconde membrane,
iii) mettre en contact la lignée cellulaire cible avec la membrane contiguë et infecter ainsi la lignée cellulaire cible avec l'agent EST, étant précisé que la seconde membrane provient du même type de cellule animale que la lignée cellulaire cible.

2. Procédé pour infecter une lignée cellulaire cible avec un agent EST comprenant les étapes consistant à :
i) former une préparation membranaire contenant un agent EST provenant d'une première espèce animale dans une première membrane, et mélanger cette préparation membranaire avec une seconde membrane provenant d'une seconde espèce animale, la première et la seconde espèce animale étant différentes,
ii) former une membrane contiguë comprenant l'agent EST et la seconde membrane,
iii) mettre en contact la lignée cellulaire cible avec la membrane contiguë et infecter ainsi la lignée cellulaire cible avec l'agent EST, étant précisé que la seconde membrane provient de la même espèce animale que la lignée des cellulaire cible.

3. Procédé conforme à la revendication 1,
selon lequel
l'agent EST provenant du premier type de cellule animale et la seconde membrane proviennent d'espèces animales différentes.

4. Procédé conforme à la revendication 2,
selon lequel
l'agent EST provenant de la première espèce animale et la seconde membrane proviennent de types de cellules animales différents.

5. Procédé conforme à l'une des revendications précédentes, dans lequel
l'EST est choisi dans le groupe formé par les CJD, vCJD, CJD sporadique, CJD familiale, CJD iatrogénique, ESB, ESB, ovine et CWD.

6. Procédé conforme à l'une des revendications précédentes,
selon lequel
la source de l'agent EST est choisie dans le groupe formé par les tissus lymphatiques (par exemple l'amygdale, l'appendice et l'amygdale rectal) les tissus neuronaux (par exemple le cerveau, le système nerveux central) et le sang (par exemple les lymphocytes).

7. Procédé conforme à l'une des revendications précédentes,
selon lequel
la lignée cellulaire cible est choisie dans le groupe formé par les cellules neuronales (par exemple les cellules du neuroblastome) et le sang (par exemple les lymphocytes.

8. Procédé conforme à l'une des revendications précédentes,
selon lequel
la seconde membrane et la lignée cellulaire cible proviennent d'une espèce animale choisie dans le groupe formé par l'homme, le mouton, la vache, la souris et le cerf.

9. Procédé conforme à l'une des revendications précédentes,
selon lequel
la seconde membrane et la lignée cellulaire cible proviennent d'un type de cellules choisi dans le groupe formé par les cellules du neuroblastome (par exemple NB69, SK-N-SH, et Kelly).

10. Procédé conforme à l'une des revendications précédentes,
selon lequel
la lignée des cellulaire cible et la cellule à partir de laquelle l'agent EST est obtenu, proviennent de la même espèce animale, de préférence du groupe formé par l'homme, le mouton, la vache, la souris et le cerf.

11. Procédé conforme à l'une des revendications précédentes,
selon lequel
la lignée cellulaire cible et la cellule à partir de laquelle l'agent EST est obtenu, sont du même type, et proviennent de préférence du groupe formé par les cellules du neuroblastome (par exemple NB69, SK-N-SH, et Kelly).

12. Procédé conforme à la revendication 2,
selon lequel
la seconde membrane et les cellules à partir desquelles l'agent EST est obtenu, proviennent du même type de cellules, de préférence du groupe formé par les cellules du neuroblastome (par exemple NB69, SK-N-SH, et Kelly).

13. Procédé conforme à l'une des revendications 1 à 4,
selon lequel
l'agent EST est choisi dans le groupe formé par les CJD, vCJD, CJD sporadique, CJD familiale, CJD iatrogénique, la lignée cellulaire cible et la seconde membrane provenant d'une espèce non-humaine (par exemple de la souris, du rat, de la vache, du mouton ou du cerf).

14. Procédé conforme à l'une des revendications 1 à 4,
selon lequel
l'agent EST est l'ESB, la lignée cellulaire cible et la seconde membrane provenant d'une espèce non-bovine (par exemple de l'homme, de la souris, du rat, du mouton ou du cerf).

15. Procédé conforme à l'une des revendications 1 à 4,
selon lequel
l'agent EST est la CWD, la lignée cellulaire cible et la seconde membrane provenant d'une espèce différente du cerf (par exemple de l'homme, de la souris, du rat, de la vache ou du mouton).

16. Procédé conforme à l'une des revendications 1 à 4,
selon lequel
l'agent EST est l'ESB ovine ou tremblante du mouton (scrapie), la lignée cellulaire cible et la seconde membrane provenant d'une espèce non-ovine (par exemple de l'homme, de la souris, du rat, de la vache ou du cerf).

17. Procédé conforme à l'une des revendications précédentes,
selon lequel
la première ou la seconde membrane est une membrane microsomale.
